# EUROPEAN PATENT SPECIFICATION

(11) **EP 2 846 778 B1**
(45) Date of publication and mention of the grant of the patent: **25.04.2018**
(21) Application number: 13722251.9
(22) Date of filing: 03.05.2013
(51) Int. Cl.: A61K 9/20, A61K 31/4709, A61P 31/14

(54) **ORAL SOLID DOSAGE FORMULATION OF 1,1-DIMETHYLETHYL [(1S)-1-{[(2S,4R)-4-(7-CHLORO-4METHOXYISOQUINOLIN-1-YLOXY)-2-({(1R,2S)-1-[(CYCLOPROPYLSULFONYL)CARBAMOYL]-2-ETHENYLCYCLOPROPYL}CARBAMOYL)PYRROLIDIN-1-YL]CARBONYL}-2,2-DIMETHYLPROPYL]CARBAMATE**
ORALE FESTE VERABREICHUNGSFORM VON 1,1-DIMETHYLETHYL-[(1S)-1-{[(2S,4R)-4-(7-CHLOR-4METHOXYISOCHINOLIN-1-YLOXY)-2-({(1R,2S)-1-[(CYCLOPROPYLSULFONYL)CARBAMOYL]-2-ETHENYLCYCLOPROPYL}CARBAMOYL)PYRROLIDIN-1-YL]CARBONYL}-2,2-DIMETHYLPROPYL]CARBAMAT
FORMULATION POSOLOGIQUE SOLIDE ORALE DE [(1S)-1-{[(2S,4R)-4-(7-CHLORO-4-MÉTHOXYISOQUINOLIN-1-YLOXY)-2-({(1R,2S)-1-[(CYCLOPROPYLSULFONYL)CARBAMOYL]-2-ÉTHÉNYLCYCLOPROPYL}CARBAMOYL)PYRROLIDIN-1-YL]CARBONYL}-2,2-DIMÉTHYLPROPYL]CARBAMATE DE 1,1-DIMÉTHYLÉTHYLE

(30) Priority: 07.05.2012 US 201261643486 P
(43) Date of publication of application: 18.03.2015
(73) Proprietor: Bristol-Myers Squibb Holdings Ireland, Steinhausen (CH)
(72) Inventor: HAMEY, Rhye, New Brunswick, New Jersey 08903 (US); PANDEY, Preetanshu, New Brunswick, New Jersey 08903 (US); BINDRA, Dilbir S., New Brunswick, New Jersey 08903 (US); VEMAVARAPU, Chandra, Pennington, New Jersey 08534 (US); PERRONE, Robert Kevin, New Brunswick, New Jersey 08903 (US)
(74) Representative: Vögeli-Lange, Regina
(86) International application number: PCT/US2013/039378
(87) International publication number: WO 2013/169577

(56) References cited:
- KAZUAKI CHAYAMA ET AL: "Dual therapy with the nonstructural protein 5A inhibitor, daclatasvir, and the nonstructural protein 3 protease inhibitor, asunaprevir, in hepatitis C virus genotype 1b-infected null responders", HEPATOLOGY, vol. 55, no. 3, 30 March 2012 (2012-03-30) , pages 742-748, XP055071008, ISSN: 0270-9139, DOI: 10.1002/hep.24724
- FEIYAN JIN ET AL: "Tabletability assessment of conventional formulations containing Vitamin E tocopheryl polyethylene glycol succinate", INTERNATIONAL JOURNAL OF PHARMACEUTICS, ELSEVIER BV, NL, vol. 389, no. 1, 10 January 2010 (2010-01-10), pages 58-65, XP028308321, ISSN: 0378-5173, DOI: 10.1016/J.IJPHARM.2010.01.017 [retrieved on 2010-01-18]
- C. PASQUINELLI ET AL: "Single- and Multiple-Ascending-Dose Studies of the NS3 Protease Inhibitor Asunaprevir in Subjects with or without Chronic Hepatitis C", ANTIMICROBIAL AGENTS AND CHEMOTHERAPY, vol. 56, no. 4, 1 April 2012 (2012-04-01), pages 1838-1844, XP055071012, ISSN: 0066-4804, DOI: 10.1128/AAC.05854-11
- AUNGST B J: "INTESTINAL PERMEATION ENHANCERS", JOURNAL OF PHARMACEUTICAL SCIENCES, AMERICAN PHARMACEUTICAL ASSOCIATION, WASHINGTON, US, vol. 89, no. 4, 1 April 2000 (2000-04-01), pages 429-442, XP000928598, ISSN: 0022-3549, DOI: 10.1002/(SICI)1520-6017(200004)89:4<429::A ID-JPS1>3.0.CO;2-J

## Description

### FIELD OF THE INVENTION

The invention relates generally to an oral solid dosage formulation for poorly water soluble pharmaceutical compounds that exhibit significant food effect on oral bioavailability. In particular, the invention relates to a new oral solid dosage formulation of Asunaprevir, 1,1-dimethylethyl [(1*S*)-1-{[(2*S*,4*R*)-4-(7-chloro-4methoxyisoquinolin-1-yloxy)-2-({(1*R*,2*S*)-1-[(cyclopropylsulfonyl)carbamoyl]-2-ethenylcyclopropyl}carbamoyl)pyrrolidin-1-yl]carbonyl}-2,2-dimethylpropyl]carbamate, to provide a total blood plasma concentration profile that is greater than the total blood plasma concentration of an orally administered solution comprising the compound, and for the treatment and/or inhibition of the hepatitis C virus and infections caused thereby.

### BACKGROUND OF THE INVENTION

The compound of formula (I), Asunaprevir, 1,1-dimethylethyl [(1*S*)-1-{[(2*S*,4*R*)-4-(7-chloro-4methoxyisoquinolin-1-yloxy)-2-({(1*R*,2*S*)-1-[(cyclopropylsulfonyl)carbamoyl]-2-ethenylcyclopropyl}carbamoyl)pyrrolidin-1-yl]carbonyl}-2,2-dimethylpropyl]carbamate, is a selective NS3 protease inhibitor and is useful in the treatment of the hepatitis C virus (HCV)

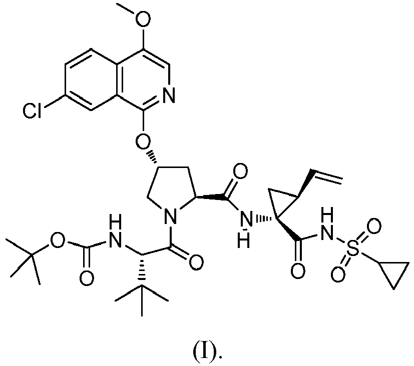

The compound of formula (I) and its preparation has been previously described in U.S. Patent No. 6,995,174, issued February 7, 2006, U.S. Patent No. 7,449,479, issued November 11, 1988, and U.S. Patent No. 7,915,291 which issued March 29, 2011. Asunaprevir is also described in Chayama et al. (Hepatology, vol. 55, n.3, March 2012, pages 742-748) in the context of an oral combination therapy of daclatasvir (30 mg tablets) and asunaprevir (200 mg tablets).

Asunaprevir is a poorly water soluble compound that in various formulations has exhibited a significant food effect. The present invention provides an oral solid dosage formulation that can be manufactured through conventional wet granulation technology, is amenable to fixed dose combinations, mitigates the food effect, and increases the bioavailability of the compound in the fasted state.

Clinical studies of Asunaprevir have shown that the current formulation needs to be improved due to both low bioavailability and food effects observed in the trials. It has surprisingly been found that an oral solid dosage formulation employing d-alpha tocopheryl polyethylene glycol 1000 succinate (Vitamin ETPGS) results in enhanced bioavailability as well as mitigation of the food effects observed in the current clinical formulation as well as other proposed formulations.

Vitamin E TPGS is well known as a bioavailability enhancer. Due to its low melting point, there is limited publication with it being used in solid dosage formulations. For this formulation, Vitamin E TPGS is used as a binder, surfactant, solubilizer, lubricant, and bioavailability enhancer. While not surmising the mechanism involved, it was surprisingly and unexpectedly found that when Vitamin E TPGS is combined with the other surfactant(s) used in the formulation, such as poloxamer and sodium lauryl sulfate, that the formulation was able to mitigate the food effect observed with other formulations. Vitamin E TPGS administered by itself or in combination with other surfactants is not known to mitigate a food effect.

Thus, it was observed that using only about 4-5% Vitamin E TPGS resulted in a threefold increase in bioavailability as well as significant mitigation of the food effect.

Thus, it is an object of the invention to prepare oral solid dosage formulations that will increase the bioavailability of an API with low solubility, such as Asunaprevir, and mitigate any observed food effects.

### SUMMARY OF THE INVENTION

The present invention is directed to an oral solid dosage formulation of the compound of formula (I).The formulation can be used to provide a total blood plasma concentration profile that is greater than the total blood plasma concentration of an orally administered solution comprising the compound, and for treating and/or inhibiting the hepatitis C virus using said formulation.

### DETAILED DESCRIPTION OF THE INVENTION

In one aspect, there is disclosed an oral solid dosage formulation of the compound I of the formula

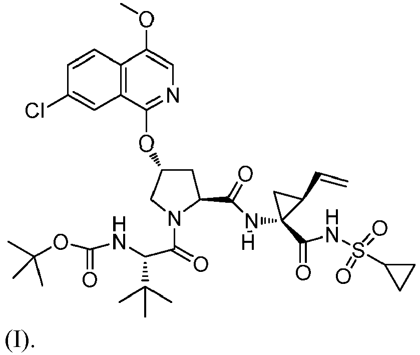

In another aspect, there is disclosed an oral solid dosage formulation comprising at least one pharmaceutical agent comprising Compound I of the formula 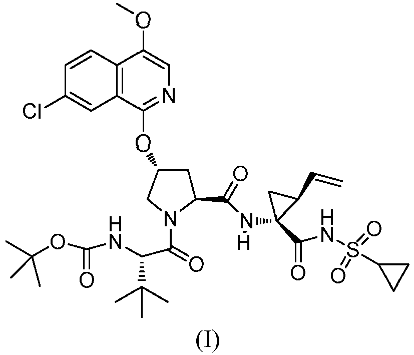 in the range of 30-80% w/w and a bioavailability enhancer is included in the range of 2-20% w/w of the total formulation.

In an embodiment of this aspect, there is disclosed the oral solid dosage formulation wherein a surfactant is included in the range of 2-10%.

In another embodiment of this aspect, there is disclosed the oral solid dosage formulation wherein the active pharmaceutical agent is included in the formulation in an amount of at least about 40% w/w.

In another embodiment of this aspect, there is disclosed the oral solid dosage formulation wherein the active pharmaceutical agent is included in the formulation in an amount of at least about 50% w/w.

In another embodiment of this aspect, there is disclosed the oral solid dosage formulation wherein the active pharmaceutical agent is included in the formulation in an amount of at least about 60% w/w.

In another embodiment of this aspect, there is disclosed the oral solid dosage formulation wherein the formulation is a tablet.

In another embodiment of this aspect, there is disclosed the oral solid dosage formulation wherein the formulation is a wet granulated tablet.

In another aspect, there is disclosed a method of administering an oral solid dosage formulation comprising orally administering to a fasted mammalian subject the formulation comprising Compound I having the formula 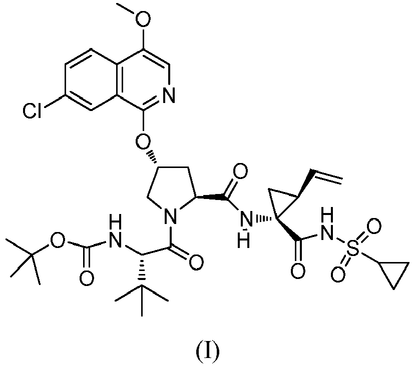 to provide a blood plasma concentration profile after an initial dose of the formulation with a Cmax of Compound I after an initial dose of the formulation that is at least greater than about 25% of the Cmax of an orally administered solution comprising Compound I.

In an embodiment of this aspect, there is disclosed the method wherein the Cmax of the formulation is at least or greater than about 30% of the Cmax of an orally administered solution.

In another embodiment of this aspect, there is disclosed the method wherein the Cmax of the formulation is at least or greater than about 35% of the Cmax of an orally administered solution.

In another embodiment of this aspect, there is disclosed the method wherein the Tmax is at least about 3 hours.

In another embodiment of this aspect, there is disclosed the method wherein the formulation is a tablet.

In another embodiment of this aspect, there is disclosed the method wherein the formulation is a wet granulated tablet.

In a first aspect of the invention, there is disclosed an oral solid dosage formulation of Compound I of the formula 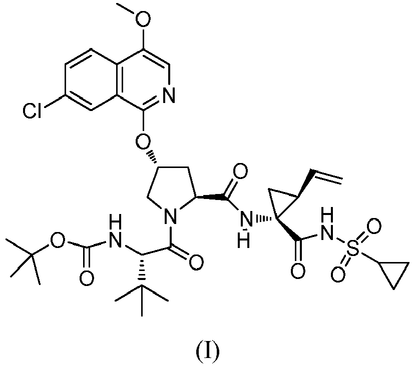 which comprises the active pharmaceutical ingredient and one or more bioavailability enhancer, filler, surfactant, disintegrant, and lubricant and optionally one or more binder and/or glidant wherein the active pharmaceutical ingredient is included in the range of 30-80% w/w, the filler is included in the range of 15-65% w/w, the binder is included in the range of 0-10% w/w, the disintegrant is included in the range of 1-20% w/w, the surfactant is included in the range of 2-10% w/w, the glidant is included in the range of 0-10% w/w, the bioavailability enhancer is included in the range of 2-20% w/w and the lubricant is included in the range of 0.25-2.0% w/w of the total formulation and the bioavailability enhancer is vitamin E TPGS and the surfactant is selected from the group consisting of poloxamer, sodium lauryl sulfate and polysorbate 80.

In an embodiment of this aspect of the invention, there is disclosed the formulation wherein the API is included in the range of 40-70% w/w, the filler is included in the range of 20-50% w/w, the binder is included in the range of 0-5% w/w, the disintegrant is included in the range of 5-15% w/w, the surfactant is included in the range of 3-6%, the glidant is included in the range of 0-5%, the bioavailability enhancer is included in the range of 4-6% and the lubricant is included in the range of 0.35-1.0% w/w.

In another embodiment of this aspect of the invention, there is disclosed the formulation wherein the API is included in about 50% w/w, the filler is included in about 25-35% w/w, the binder is included in the range of 0-2% w/w, the disintegrant is included in the range of 10-12% w/w, the surfactant is included in about 4%, the glidant is included in the range of 0-3%, the bioavailability enhancer is included in about 4.67% and the lubricant is included in about 0.5% w/w.

In another embodiment of this aspect of the invention, there is disclosed the formulation wherein the fillers and disintegrants are intragranular and extragranular.

In another embodiment of this aspect of the invention, there is disclosed the formulation wherein the fillers are selected from lactose monohydrate and microcrystalline cellulose.

In another embodiment of this aspect of the invention, there is disclosed the formulation wherein the binder is hydroxypropyl cellulose (HPC), polyvinyl pyrrolidone (PVP), starch or hydroxypropyl methylcellulose (HPMC).

In another embodiment of this aspect of the invention, there is disclosed the formulation wherein the disintegrants are selected from croscarmellose sodium, crospovidone, starch and sodium starch glycolate.

In another embodiment of this aspect of the invention, there is disclosed the formulation wherein the lubricant is magnesium stearate or sodium stearyl fumarate.

In another embodiment of this aspect of the invention, there is disclosed the formulation wherein the glidant is colloidal silicon dioxide or silicon dioxide.

In another embodiment of this aspect of the invention, there is disclosed the formulation wherein the API is Asunaprevir, the fillers are microcrystalline cellulose and/or lactose monohydrate, the binder is hydroxypropyl cellulose and/or PVP, the disintegrant is croscarmellose sodium, the surfactant is poloxamer and/or sodium lauryl sulfate, the bioavailability enhancer is Vitamin E TPGS, the glidant is colloidal silicon dioxide and the lubricant is magnesium stearate.

In another embodiment of this aspect of the invention, the oral solid dosage formulation of Compound I of the formula 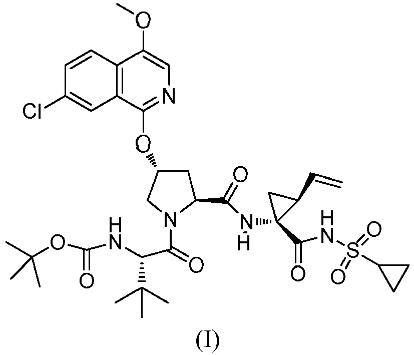 is prepared by a wet granulation process.

In an embodiment of this aspect there is disclosed the oral solid dosage formulation prepared by a wet granulation process wherein the API is included in about 50% w/w, the filler is included in about 25-35% w/w, the binder is included in the range of 0-2% w/w, the disintegrant is included in the range of 10-12% w/w, the surfactant is included in about 4%, the glidant is included in the range of 0-3%, the bioavailability enhancer is included in about 4.67% and the lubricant is included in about 0.5% w/w.

In another aspect, there is disclosed a method of administering an oral solid dosage formulation comprising orally administering to a fasted mammalian subject the formulation comprising Compound I having the formula 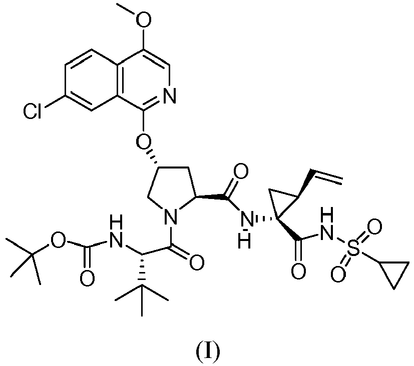 to provide a total blood plasma concentration profile of Compound I as measured by AUC at 24 hours after an initial dose of the formulation that is at least greater than about 15% of the total blood plasma concentration as measured by AUC at 24 hours of an initial dose of an orally administered solution comprising Compound I.

In an embodiment of this aspect, there is disclosed the method wherein the AUC is at least greater than about 20% of the AUC at 24 hours of the solution when orally administered regardless if the subject is fasted or fed.

In another embodiment of this aspect, there is disclosed the method wherein the AUC is at least greater than about 25% of the AUC at 24 hours of the solution when orally administered regardless if the subject is fasted or fed.

In another embodiment of thisaspect, there is disclosed the method wherein the formulation is a tablet.

In another embodiment of this aspect, there is disclosed the method wherein the formulation is a wet granulated tablet.

In another embodiment of this aspect, there is disclosed the method wherein the formulation comprises Vitamin E TPGS.

In another embodiment of this aspect, there is disclosed the method wherein the formulation comprises at least 3% by weight Vitamin E TPGS.

In another embodiment of this aspect, there is disclosed the method wherein the formulation comprises at least 4% by weight Vitamin E TPGS.

In another embodiment of this aspect, there is disclosed the method wherein the formulation comprises at least 5% by weight Vitamin E TPGS.

In another embodiment of this aspect, there is disclosed the method wherein the formulation comprises Vitamin E TPGS and at least one surfactant.

In another embodiment of this aspect, there is disclosed the method wherein the formulation comprises Vitamin E TPGS and at least one surfactant selected from the group consisting of poloxamer and sodium lauryl sulfate.

In another aspect, there is disclosed a method of administering an oral solid dosage formulation comprising orally administering to a fasted mammalian subject the formulation comprising at least one poorly soluble active pharmaceutical agent to provide a total blood plasma concentration profile as measured by AUC at 24 hours after an initial dose of the formulation that is at least greater than about 15% of the total blood plasma concentration as measured by AUC at 24 hours of an initial dose of an orally administered solution comprising the at least one active pharmaceutical agent.

In an embodiment of this aspect, there is disclosed a method wherein the active pharmaceutical agent exhibits a significant food effect.

In another embodiment of this aspect, there is disclosed a method wherein the at least one active pharmaceutical agent is included in the range from about 30 to about 80% w/w.

In another embodiment of this aspect, there is disclosed a method wherein the formulation further comprises a bioavailability enhancer is included in the range from about 2 to about 20% and optionally comprises a surfactant in the range from about 2 to about 10%.

In another aspect of the invention, there is disclosed the oral solid dosage formulation of the invention for use in the treatment of an hepatitis C virus infection.

There is described a method of inhibiting the HCV virus, comprising the step of administering to a subject in need thereof an effective amount of the oral solid dosage formulation of the invention.

There is described a method of treating an HCV infection, comprising the step of administering to a subject in need thereof an effective amount of the oral solid dosage formulation prepared by a wet granulation process.

There is described a method for treating an HCV infection, comprising administering to a patient in need thereof a therapeutically effective amount of a formulation of the present invention.

There is described a method for preparing the formulation of the invention using a wet granulation process.

In another aspect of the present invention, there is provided the use of the formulation of the present invention in therapy.

There is described the use of the formulation of the present invention in the preparation of a medicament for the treatment of the hepatitis C virus.

There is described a high drug load formulation of Compound I which mitigates food effects shown in other formulations comprising Compound I.

### DEFINITIONS

"Therapeutically effective amount" is intended to include an amount of a compound of the present invention alone or an amount of the combination of compounds claimed or an amount of a compound of the present invention in combination with other active ingredients effective to inhibit or effective to treat or prevent HCV infection.

As used herein, the terms "treating" or "treatment" refer to the treatment of a disease-state in a mammal, particularly in a human, and include: (a) preventing the disease-state from occurring in a mammal, in particular, when such mammal is predisposed to the disease-state but has not yet been diagnosed as having it; (b) inhibiting the disease-state, *i.e*., arresting it development; and/or (c) relieving the disease-state, *i.e.,* causing regression of the disease state.

As used herein, the term active pharmaceutical ingredient (API) or pharmaceutical agent refers to Asunaprevir (Compound I).

As used herein, the term "filler" refers to any pharmaceutically acceptable inert material or composition added to a formulation to add bulk. Suitable filler include for example, lactose monohydrate and microcrystalline cellulose.

As used herein, the term "disintegrant" refers to materials added to the composition to help it break apart and release the medicaments. Examples of disintegrants include, but are not limited to, non-saccharide water soluble polymers, such as cross-linked povidone. Other disintegrants that can be used include, for example, croscarmellose sodium, starch and sodium starch glycolate.

As used herein, the term "lubricant" refers to any pharmaceutically acceptable agent which reduces surface friction, lubricates the surface of the granule, and decreases the tendency to build up static electricity. Lubricants can also play a related role in improving the compression process by reducing the tendency of the material to adhere to the surface of compression tools. Thus, lubricants can serve as anti-adherents. Examples of suitable lubricants are magnesium stearate, stearic acid or other hydrogenated vegetable oil or triglycerides.

As used herein, the term "binder" refers to any pharmaceutically acceptable compound or composition that can help bind primary powder particles into agglomerates. Examples of suitable binding agents include, but are not limited to, hydroxypropyl cellulose (HPC), polyvinyl pyrrolidone (PVP), starch or hydroxypropyl methylcellulose (HPMC).

As used herein, the term "surfactant" refers to any pharmaceutically acceptable agent short for "surface acting agent" acted to modify a material or materials' physical or chemical properties such as wetability, solubility, stability, and miscibility.

As used herein, the term "bioavailability enhancer" refers to any pharmaceutically acceptable agent which is used to increase the absorption or solubility of a drug leading to an increase in bioavailability.

As used herein, the term "solubilizer" refers to any pharmaceutically acceptable agent which increases the solubility of a drug in a particular solvent or solution.

### EXAMPLES

The invention is further understood by reference to the following examples, which are intended to be purely exemplary of the invention. The present invention is not limited in scope by the exemplified embodiments, which are intended as illustrations of single aspects of the invention only. Various modifications of the invention in addition to those described herein will become apparent to those skilled in the art.

Asunaprevir can be prepared following the procedure described in U.S. Patent No. 6,995,174, issued February 7, 2006.

The manufacturing process of this formulation generally involves mixing the drug with dry powder excipients, such as with one or more binder, disintegrant, and filler. Water is then added to this premix while it is being continuously mixed in a mixer such as a high shear granulator, fluid bed granulator or other manufacturing process where a solution is used for granulating a product. Alternatively, the binder may not be added as a dry powder to the premix, but dissolved in water. Addition of water with continuous mixing leads to the formation of granules, or granulation. The granules are then dried in dry, hot air using equipment and processes such as fluid bed drying or tray drying. Once dried, the granules may be milled using equipment such as Comill or Fitzmill to reduce and/or reduce the width of distribution of particle size of granules. The granules are then mixed with extra-granular ingredients, which may include optionally one or more of disintegrant, filler, glidant, and lubricant. The final blend is then compressed on a tablet press into core tablets. The core tablets may optionally be coated with a nonfunctional film coat.

Further examples of formulating techniques can be found in Pandey et al., Pharmaceutical Development and Technology, 2013; 18(1): 296-304.

### Example 1

### Wet Granulation

The following is a typical procedure for manufacturing the wet granulated solid formulation of the invention.

Vitamin E TPGS is dissolved into water, by adding up to 20% w/w solution Vitamin E TPGS to water while agitating. The API (prepared as described in U.S. Patent No. 6,995,174), filler, surfactant/solubilizer (*e.g.,* poloxamer), disintegrant (*e.g.,* croscarmellose sodium), and binder (*e.g*., polyvinylpyrrolidone) if present, are blended together. The granulation is performed by adding the solution of Vitamin E TPGS to the dry blend, while being mixed in the high shear granulator.

Wet granulations are removed from the granulator, and screened as needed. The granulation is dried in a fluid bed, oven or other type of drier. The dried granulation is milled or screened as needed. The dried granulation may be mixed with one or more of the following materials, glidant(s) (*e.g.,* colloidal silicon dioxide), disintegrant(s) (*e.g.,* croscarmellose sodium), lubricant(s) (*e.g.,* magnesium stearate), and/or filler(s) (*e.g.,* microcrystalline cellulose) to complete the formulation.

The following 5 Tables disclose typical formulations prepared using the above procedure. Table 1 to 4 are according to the present invention.

**Table 1**

| Ingredient | mg/tablet | %(w/w) |
|---|---|---|
| Compound I | 200 | 50 |
| Lactose Monohydrate | 60 | 16 |
| Microcrystalline Cellulose | 58 | 14.5 |
| Croscarmellose Sodium | 46 | 11.5 |
| Vitamin E TPGS | 18 | 4.5 |
| Poloxamer | 16 | 4 |
| Magnesium Stearate | 2 | 0.5 |
| | | |
| Total | 400 | 100 |

**Table 2**

| Ingredient | mg/tablet | %(w/w) |
|---|---|---|
| Compound I | 200 | 50 |
| Lactose Monohydrate | 56.76 | 14.19 |
| Microcrystalline Cellulose | 60.56 | 15.14 |
| Croscarmellose Sodium | 46 | 11.5 |
| Vitamin E TPGS | 18.68 | 4.67 |
| Sodium Lauryl Sulfate | 16 | 4 |
| Magnesium Stearate | 2 | 0.5 |
| | | |
| Total | 400 | 100 |

**Table 3**

| Ingredient | mg/tablet | %(w/w) |
|---|---|---|
| Compound I | 200 | 50 |
| Lactose Monohydrate | 52.76 | 13.19 |
| Microcrystalline Cellulose | 56.56 | 14.14 |
| Croscarmellose Sodium | 46 | 11.5 |
| Vitamin E TPGS | 18.68 | 4.67 |
| Sodium Lauryl Sulfate | 16 | 4 |
| Polyvinylpyrrolidone | 8 | 2 |
| Magnesium Stearate | 2 | 0.5 |
| | | |
| Total | 400 | 100 |

**Table 4**

| Ingredient | mg/tablet | %(w/w) |
|---|---|---|
| Compound I | 200 | 50 |
| Lactose Monohydrate | 52.76 | 13.19 |
| Microcrystalline Cellulose | 56.56 | 14.14 |
| Croscarmellose Sodium | 46 | 11.5 |
| Vitamin E TPGS | 18.68 | 4.67 |
| Sodium Lauryl Sulfate | 16 | 4 |
| Hydroxypropyl cellulose | 8 | 2 |
| Magnesium Stearate | 2 | 0.5 |
| | | |
| Total | 400 | 100 |

**Table 5**

| Ingredient | mg/tablet | %(w/w) |
|---|---|---|
| Compound I | 200 | 50 |
| Lactose Monohydrate | 64 | 16 |
| Microcrystalline Cellulose | 64 | 16 |
| Croscarmellose Sodium | 46 | 11.5 |
| Poloxamer | 16 | 4 |
| Polyvinylpyrrolidone | 8 | 2 |
| Magnesium Stearate | 2 | 0.5 |
| | | |
| Total | 400 | 100 |

### Example 2

### Bioequivalence Study

A study was conducted to compare conventional tablet dosage forms. The goal of the study was to assess the *in vivo* performance of various prototypes of conventional tablet dosage forms (wet and dry granulated tablets) in a dog model.

The following five formulations were tested:
1. Phase 2 Clinical (dry granulated (DG)) tablet 2x200 mg (reference formulation).
2. Wet granulated (WG) Vitamin E TPGS/poloxamer tablet: 50% w/w Compound I+ 4.67% w/w Vitamin E TPGS+ 4% w/w poloxamer+ 14.33% w/w microcrystalline cellulose+ 15% lactose monohydrate+ 11.5% w/w croscarmellose sodium+ 0.5% w/w magnesium stearate; 2 x 200 mg (Corresponds to the formulation shown above in Table 1).
3. WG poloxamer tablet: 50% w/w Compound I+ 2% w/w polyvinyl pyrrolidone + 4% w/w poloxamer + 16% microcrystalline cellulose+ 16% w/w lactose monohydrate+ 11.5% w/w croscarmellose sodium+ 0.5% w/w magnesium stearate; 2 x 200 mg (Corresponds to the reference formulation shown above in Table 5).
4. Amorphous API tablet (reference formulation): 50% w/w Compound I + 41.25% w/w microcrystalline cellulose + 5% w/w croscarmellose sodium + 2% w/w silicon dioxide+ 1% w/w sodium lauryl sulphate+ 0.75% w/w magnesium stearate; 2 x 200 mg.
5. Solution Formulation: Polyethylene glycol 400:EtOH: polysorbate 80 (50:35:15), 20 mg/mL (reference formulation).

The protocol used was as follows:

Crossover in 4 fasted, pentagastrin-treated, male dogs (∼10 kg), 400 mg dose flushed with 50 ml water. For food-effect studies in the fed state, dogs were given 50 ml high-fat meal substitute instead of water.

The following was observed (as shown in Table 6 below):

**Table 6**

| PK Parameters for Wet(WG) and Dry Granulated (DG) Formulations in a Dog Model | | | | | | | | | | | |
|---|---|---|---|---|---|---|---|---|---|---|---|
| | | Cmax | | | %CV | Tmax | AUC | | Relative BA | | |
| | | Mean | SD | Relative Cmax | % Std Dev | median | Mean | SD | Mean | SD | %CV |
| DG tablet | fasted | 8693.61 | 585.82 | 20.16 | | 2.50 | 40519.42 | 8795.62 | 8.04 | 1.75 | 21.71 |
| WG TPGS/Pluronic | fasted | 14381.70 | 3776.03 | 33.35 | 26.26 | 3.00 | 104507.16 | 28685.28 | 20.73 | 5.69 | 27.45 |
| WG Pluronic | fasted | 8944.45 | 6743.93 | 20.74 | 75.40 | 3.00 | 45711.73 | 38370.23 | 9.07 | 7.61 | 83.94 |

- Tablet formulations in the fasted state range from 8-21% bioavailability.
- The Vitamin E TPGS/poloxamer (Pluronic) wet granulation tablet is the best performing tablet with the highest Cmax and AUC compared to the other prototypes.
- The presence of poloxamer alone does not improve performance.
- The presence of Vitamin E TPGS as a solubilizer improves performance, and also significantly reduces variability compared to the wet granulated tablet without Vitamin E TPGS.

The dog model showed that Compound I has a significant positive food effect. The effect is related to low API dissolution/high solubility in lipid environment and slower GI motility (higher Tmax). It was observed that the presence of Vitamin E TPGS in the tablet formulations appears to improve its *in vivo* performance and reduce variability compared to the wet granulated formulation that did not contain Vitamin E TPGS.

A clinical study in humans was conducted to evaluate the pharmacokinetics and effect of fasted vs. fed subjects on the oral absorption of various formulations of Compound I. Using the relative bioavailability and comparing the wet granulated(WG) tablet containing Vitamin E TPGS with a dry granulated(DG) tablet that had previously been used (reference formulation), the following results were observed:

| | Fed | Fasted | Ratio |
|---|---|---|---|
| DG Tablet | 43% | ∼10% | 4.3 |
| WG Tablet | 34% | 26% | 1.31 |

Thus, the wet granulated formulation of the invention was found to provide consistent oral bioavailability of Compound I without regard for the dietary state of the patient.

Although the foregoing invention has been described in some detail to facilitate understanding, it will be apparent that certain changes and modifications may be practiced within the scope of the appended claims. Accordingly, the described embodiments are to be considered as illustrative and not restrictive, and the invention is not to be limited to the details given herein, but may be modified within the scope of the appended claims.

## Claims

1. An oral solid dosage formulation of Compound I of the formula 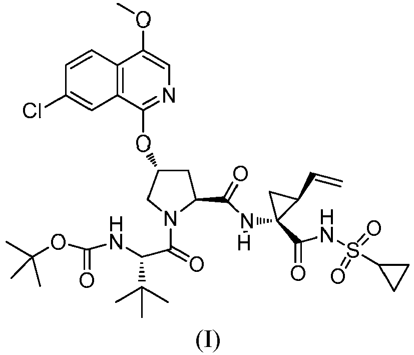 which comprises the active pharmaceutical ingredient and one or more bioavailability enhancer, filler, surfactant, disintegrant, and lubricant and optionally one or more binder and/or glidant wherein
the active pharmaceutical ingredient is included in the range of 30-80% w/w,
the filler is included in the range of 15-65% w/w,
the binder is included in the range of 0-10% w/w,
the disintegrant is included in the range of 1-20% w/w,
the surfactant is included in the range of 2-10% w/w,
the glidant is included in the range of 0-10% w/w,
the bioavailability enhancer is included in the range of 2-20% w/w and
the lubricant is included in the range of 0.25-2.0% w/w of the total formulation, **characterized in that** the bioavailability enhancer is vitamin E TPGS and the surfactant is selected from the group consisting of poloxamer, sodium lauryl sulfate and polysorbate 80.

2. The formulation of claim 1, wherein the active pharmaceutical ingredient is included in the range of 40-70% w/w, the filler is included in the range of 20-50% w/w, the binder is included in the range of 0-5% w/w, the disintegrant is included in the range of 5-15% w/w, the surfactant is included in the range of 3-6% w/w, the glidant is included in the range of 0-5% w/w, the bioavailability enhancer is included in the range of 4-6% and the lubricant is included in the range of 0.35-1.0% w/w.

3. The formulation of claim 1, wherein the active pharmaceutical ingredient is included in 50% w/w, the filler is included in the range of 25-35% w/w, the binder is included in the range of 0-2% w/w, the disintegrant is included in the range of 10-12% w/w, the surfactant is included in 4%, w/w the glidant is included in the range of 0-3% w/w, the bioavailability enhancer is included in 4.67% w/w and the lubricant is included in 0.5% w/w.

4. The formulation of claim 1, wherein the fillers are microcrystalline cellulose and/or lactose monohydrate, the binder is hydroxypropyl cellulose and/or PVP, the disintegrant is croscarmellose sodium, the surfactant is poloxamer and/or sodium lauryl sulfate, the bioavailability enhancer/solubilizer is Vitamin E TPGS, the glidant is colloidal silicon dioxide and the lubricant is magnesium stearate.

5. The formulation of claim 1, which comprises 4-5% by weight Vitamin E TPGS.

6. The formulation of claim 1 wherein the formulation is a wet granulated tablet formulation.

7. A formulation of any one of claims 1 to 6 for use as a medicament.

8. A formulation of any one of claims 1 to 6 for use in the treatment of a hepatitis C virus infection.

## Patentansprüche

1. Orale feste Dosierungsformulierung von Verbindung I der Formel 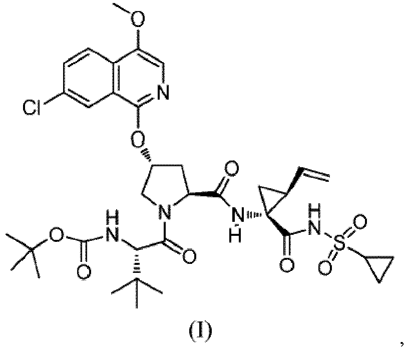 die den aktiven pharmazeutischen Inhaltsstoff und ein oder mehrere Bioverfügbarkeit-Verbesserungsmittel, Füllmittel, Tensid, Zersetzungsmittel und Schmiermittel und wahlweise ein oder mehrere Bindemittel und/oder Fließmittel umfasst, wobei
der aktive pharmazeutische Inhaltsstoff im Bereich von 30-80 Gew.-% enthalten ist,
das Füllmittel im Bereich von 15-65 Gew.-% enthalten ist,
das Bindemittel im Bereich von 0-10 Gew.-% enthalten ist,
das Zersetzungsmittel im Bereich von 1-20 Gew.-% enthalten ist,
das Tensid im Bereich von 2-10 Gew.-% enthalten ist,
das Fließmittel im Bereich von 0-10 Gew.-% enthalten ist,
das Bioverfügbarkeit-Verbesserungsmittel im Bereich von 2-20 Gew.-% enthalten ist und
das Schmiermittel im Bereich von 0,25-2,0 Gew.-% der gesamten Formulierung enthalten ist,
**dadurch gekennzeichnet, dass** das Bioverfügbarkeit-Verbesserungsmittel Vitamin E TPGS ist und das Tensid aus Poloxamer, Natriumlaurylsulfat und Polysorbat 80 ausgewählt ist.

2. Formulierung nach Anspruch 1, wobei der aktive pharmazeutische Inhaltsstoff im Bereich von 40-70 Gew.-% enthalten ist, das Füllmittel im Bereich von 20-55 Gew.-% enthalten ist, das Bindemittel im Bereich von 0-5 Gew.-% enthalten ist, das Zersetzungsmittel im Bereich von 5-15 Gew.-% enthalten ist, das Tensid im Bereich von 3-6 Gew.-% enthalten ist, das Fließmittel im Bereich von 0-5 Gew.-% enthalten ist, das Bioverfügbarkeit-Verbesserungsmittel im Bereich von 4-6 Gew.-% enthalten ist und das Schmiermittel im Bereich von 0,35-1,0 Gew.-% enthalten ist.

3. Formulierung nach Anspruch 1, wobei der aktive pharmazeutische Inhaltsstoff zu 50 Gew.-% enthalten ist, das Füllmittel im Bereich von 25-35 Gew.-% enthalten ist, das Bindemittel im Bereich von 0-2 Gew.-% enthalten ist, das Zersetzungsmittel im Bereich von 10-12 Gew.-% enthalten ist, das Tensid zu 4 Gew.-% enthalten ist, das Fließmittel im Bereich von 0-3 Gew.-% enthalten ist, das Bioverfügbarkeit-Verbesserungsmittel zu 4,67 Gew.-% enthalten ist und das Schmiermittel zu 0,5 Gew.-% enthalten ist.

4. Formulierung nach Anspruch 1, wobei die Füllmittel mikrokristalline Cellulose und/oder Lactosemonohydrat sind, das Bindemittel Hydroxypropylcellulose und/oder PVP ist, das Zersetzungsmittel Croscarmellose-Natrium ist, das Tensid Poloxamer und/oder Natriumlaurylsulfat ist, das Bioverfügbarkeit-Verbesserungsmittel/der Lösungsvermittler Vitamin E TPGS ist, das Fließmittel kolloidales Siliciumdioxid und das Schmiermittel Magnesiumstearat ist.

5. Formulierung nach Anspruch 1, die 4-5 % nach Gewicht Vitamin E TPGS umfasst.

6. Formulierung nach Anspruch 1, wobei die Formulierung eine nass granulierte Tablettenformulierung ist.

7. Formulierung nach einem der Ansprüche 1 bis 6 zur Verwendung als ein Medikament.

8. Formulierung nach einem der Ansprüche 1 bis 6 zur Verwendung bei der Behandlung einer Hepatitis-C-Virusinfektion.

## Revendications

1. Formulation dosifiée solide orale du Composé I de la formule: 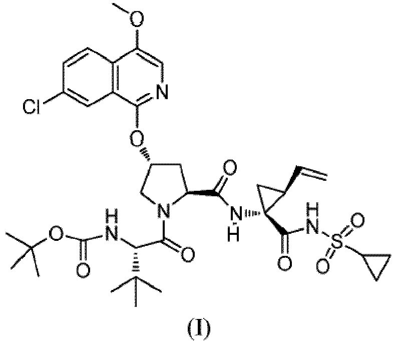 qui comprend l'ingrédient pharmaceutique actif et un ou plusieurs parmi un amplificateur de biodisponibilité, une charge, un tensioactif, un désintégrant et un lubrifiant et optionnellement un ou plusieurs parmi un liant et/ou un agent de glissement, dans laquelle
l'ingrédient pharmaceutique actif est inclus dans l'intervalle de 30-80% p/p,
la charge est incluse dans l'intervalle de 15-65% p/p,
le liant est inclus dans l'intervalle de 0-10% p/p,
le désintégrant est inclus dans l'intervalle de 1-20% p/p,
le tensioactif est inclus dans l'intervalle de 2-10% p/p,
l'agent de glissement est inclus dans l'intervalle de 0-10% p/p,
l'amplificateur de biodisponibilité est inclus dans l'intervalle de 2-20% p/p et
le lubrifiant est inclus dans l'intervalle de 0,25-2,0% p/p de la formulation totale, **caractérisée en ce que** l'amplificateur de biodisponibilité est la vitamine E TPGS et le tensioactif est choisi dans le groupe constitué par un poloxamère, le laurylsulfate de sodium et le polysorbate 80.

2. Formulation selon la revendication 1, dans laquelle l'ingrédient pharmaceutique actif est inclus dans l'intervalle de 40-70% p/p, la charge est incluse dans l'intervalle de 20-50% p/p, le liant est inclus dans l'intervalle de 0-5% p/p, le désintégrant est inclus dans l'intervalle de 5-15% p/p, le tensioactif est inclus dans l'intervalle de 3-6% p/p, l'agent de glissement est inclus dans l'intervalle de 0-5% p/p, l'amplificateur de biodisponibilité est inclus dans l'intervalle de 4-6% p/p et le lubrifiant est inclus dans l'intervalle de 0,35-1,0% p/p.

3. Formulation selon la revendication 1, dans laquelle l'ingrédient pharmaceutique actif est inclus dans 50% p/p, la charge est incluse dans l'intervalle de 25-35% p/p, le liant est inclus dans l'intervalle de 0-2% p/p, le désintégrant est inclus dans l'intervalle de 10-12% p/p, le tensioactif est inclus dans 4% p/p, l'agent de glissement est inclus dans l'intervalle de 0-3% p/p, l'amplificateur de biodisponibilité est inclus dans 4,67% p/p et le lubrifiant est inclus dans 0,5% p/p.

4. Formulation selon la revendication 1, dans laquelle les charges sont une cellulose microcristalline et/ou le monohydrate de lactose, le liant est l'hydroxypropylcellulose et/ou PVP, le désintégrant est la croscarmellose sodique, le tensioactif est un poloxamère et/ou le laurylsulfate de sodium, l'amplificateur de biodisponibilité/agent solubilisant est la vitamine E TPGS, l'agent de glissement est un dioxyde de silicium colloïdal et le lubrifiant est le stéarate de magnésium.

5. Formulation selon la revendication 1, qui comprend 4-5% en poids de vitamine E TPGS.

6. Formulation selon la revendication 1, où la formulation est une formulation de cachet granulé par voie humide.

7. Formulation selon l'une quelconque des revendications 1 à 6 pour une utilisation en tant que médicament.

8. Formulation selon l'une quelconque des revendications 1 à 6 pour une utilisation dans le traitement d'une infection par le virus de l'hépatite C.
